# EUROPEAN PATENT SPECIFICATION

(11) **EP 1 882 733 B1**
(45) Date of publication and mention of the grant of the patent: **11.07.2018**
(21) Application number: 06741894.7
(22) Date of filing: 17.05.2006
(51) Int. Cl.: C12M 1/04

(54) **TRANSESTERIFICATION PROCESS USING AN AIRLIFT REACTOR**
UMESTERUNGSVERFAHREN IN EINEM AIRLIFT REAKTOR
PROCÉDÉ DE TRANSESTÉRIFICATION DANS UN RÉACTEUR À AGITATION PAR CIRCULATION TOURNANTE

(30) Priority: 20.05.2005 CN 200510070863
(43) Date of publication of application: 30.01.2008
(73) Proprietor: Tsinghua University, Haidian District, Beijing 100084 (CN)
(72) Inventor: LI, Lilin, Department of Chemical Engineering, Beijing 100084 (CN); DU, Wei, Department of Chemical Engineering, Beijing 100084 (CN); LIU, Dehua, Department of Chemical Engineering, Beijing 100084 (CN)
(74) Representative: Ilgart, Jean-Christophe
(86) International application number: PCT/CN2006/001006
(87) International publication number: WO 2006/122498

(56) References cited:
- EP-A1- 0 310 878
- CH-A5- 688 553
- CN-A- 1 203 943
- CN-A- 1 403 579
- CN-A- 1 458 135
- CN-A- 1 583 601
- CN-U- 2 099 763
- CN-Y- 2 329 665
- CN-Y- 2 334 763
- CN-Y- 2 510 449
- CN-Y- 2 608 509
- US-A- 5 342 781
- US-A- 5 599 451

## Description

### TECHNICAL FIELD

This invention relates to biochemical and fermentation industries. In particular, it provides an airlift loop reactor without the need for external gases.

### BACKGROUND ART

An airlift loop reactor is a biological reactor which uses gases as the impetus to realize the mix and circulation of liquids. Since it is simple in structure, easy to amplify, excellent in mass and heat transfer, low in energy consumption and small in damages to cells, airlift loop reactors are more and more widely applied in biochemical and fermentation industries.

Current airlift loop reactors, which are widely used in industries, use external gases supplied from the bottom of the reactors as impetus. The gases are discharged after flowing up through the reactors. This invention proposes directly utilizing the internal gases as the impetus for the circulation of liquids inside the reactor, hence effectively reduces the cost of external gases and diminishes the production costs. Additionally, using the gases from inside makes the reaction system a closed structure, therefore effectively reducing the loss of raw materials of the reaction.

CN 1 403 579 discloses a fermentation airlift loop reactor, wherein a part of the internal gases flowing out from the top of the reactor is re-injected into the bottom of the reactor and an external gas is not needed in the process. However, the reactor of CN 1 403 579 is used for ethanol production, and thus, an additional activated carbon adsorption and desorption apparatus is disposed in the gas circulation line so as to withdraw the ethanol gas produced in the reactor, then, the remaining CO₂ is returned back to the bottom of the reactor. In other words, the gases flowing out from the top of the reactor are not returned back to the bottom of the reactor directly, but undergo a separation step.

EP 0 310 8778 discloses a loop reactor for the production of isobutyric acid, wherein the gases from the top of the reactor are returned directly to the bottom of the reactor and re-injected into the reactor together with the reactants via the feed inlet. In the reactor of EP 0 310 8778, the feed inlet also serves as the gas inlet, and external gases from outside of the reactor are used since the reactants include a large amount of gases.

CN 2510499 discloses a reactor used for plant cell culture, wherein the released gases from the cell culture are collected and re-pumped into the bottom of the reactor. However, since such a reactor is used for plant cell culture, it is necessary for the reactor to have a gas inlet for introducing gases necessary for the cell growth such as oxygen from outside.

CN 2099763 discloses an airlift bioreactor used for fermentation, wherein an external gas is ejected via the gas distribution tube into the bottom of the reactor and the gas is circulated together with the liquid within the reactor. However, once the gas is released from the liquid, it is discharged from the reactor, and no gas circulation line is provided for the recycle of the discharged gas.

### DISCLOSURE OF THE INVENTION

The object of the present invention is a process for the transesterification reaction between animal and plant oils and methanol in the presence of lipase, using an airlift loop reactor without the need for external gases.

The reactor used in the the present invention comprises: main reactor, gas circulation line, gas pump, jacket, gas inlet, gas outlet, flow guider, gas flowmeter, feed inlet and discharge opening.

Specifically, the present invention, as defined by claim 1, improves common airlift loop reactors by linking the gas outlet on top of the reactors to the gas inlet at bottom of the reactor, while installing a gas pump in the gas circulation line, hence directly using gases inside the reaction system as the impetus for the circulation. Gases are directed by the gas circulation line back to the bottom of the reactor after flowing out from the top of the reactor, re-ejected into the reactor by the gas pump and used as circulation impetus again.

Gases that are used as the circulation impetus in this invention include: gases generated during the reaction, gases participated in the reaction and volatile components.

If the circulation impetus gases are generated during the reaction, then a by-pass gas line, with a one-way valve installed on it to adjust the pressure, could be attached on the gas circulation line.

Comparing to traditional airlift loop reactors using external gases as the impetus, using internal gases as the circulation impetus effectively reduces the costs of external gases and the production cost, also the loss of raw materials of the reaction is reduced. The reactor used in the present invention is applicable for any reaction system that has gases (including gases participated in reaction or gases generated during reaction or with existence of volatile components) in it, such as biochemical reactors and enzyme reactors, including airlift loop reactors used for wastewater treatments and other environmental protection projects.

In particular, the present invention involves an airlift loop reactor for preparing biodiesel through the transesterification reaction between animal and plant oils and methanol under the catalysis of lipase, wherein volatilized methanol serves as the impetus for circulation of liquids in the reactor. This invention effectively reduces the consumption of external gases and therefore has significant economic implications and good prospects for industrial applications.

### BRIEF DESCRIPTION OF THE DRAWINGS

Fig. 1 shows the structure of an airlift loop reactor that can be used in the present invention.
Fig. 2 shows the structure of another airlift loop reactor that can be used in the present invention.
Fig. 3 shows the partial enlarged view and bottom profile of an airlift loop reactor that can be used in the present invention.

### BEST MODE FOR CARRYING OUT THE INVENTION

Below is a further explanation on working principles and concrete structures of the airlift loop reactor that can be used in the process of the present invention with reference to the attached figures.

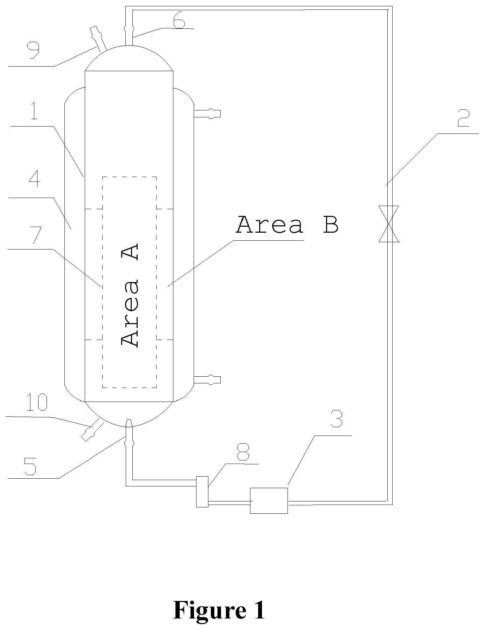

As shown in Figure 1, the reactor of the present invention mainly comprises: main reactor 1, gas circulation line 2, gas pump 3, jacket 4, gas inlet 5, gas outlet 6, flow guider 7, gas flowmeter 8, feed inlet 9 and discharge opening 10. The upper and lower ends of the gas circulation line 2, in which a gas pump 3 is installed, respectively connect to the top and bottom of the reactor. Gases are directed by the gas circulation line 2 back to the bottom of the reactor after flowing out from the top of the reactor, and re-ejected into the reactor by the gas pump 3 and used as circulation impetus again.

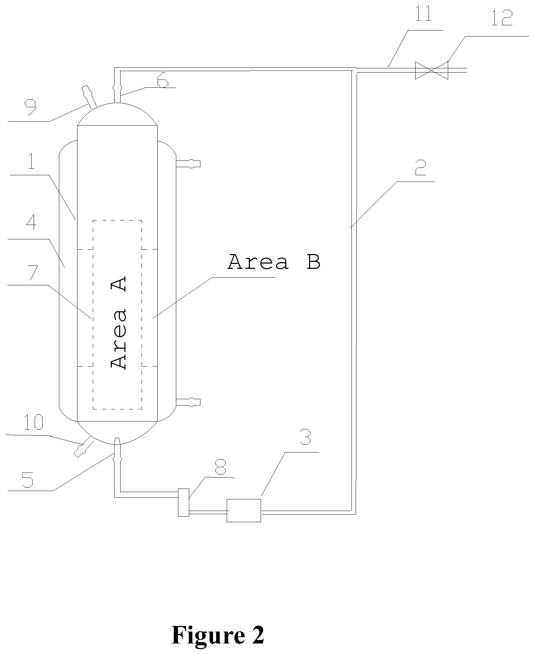

Figure 2 shows another embodiment of this invention, wherein continuously generated gases during the reaction are used as the circulation impetus. A by-pass gas line 11, with a one-way valve 12 installed on it to adjust the pressure, is attached on the gas circulation line 2.

### Example

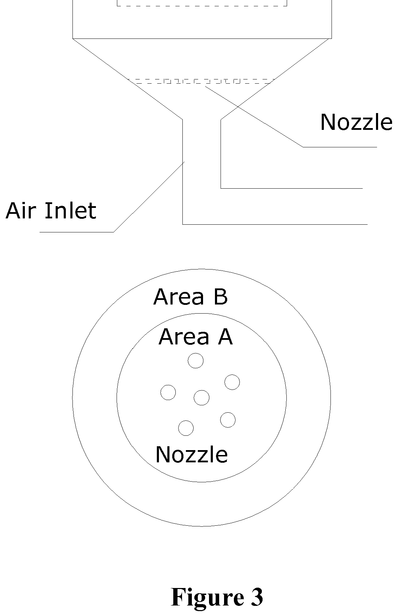

As an example of the present invention, an airlift loop reactor without the need for external gases was provided, as shown in Fig. 1 and Fig. 3. This reactor, with a height of 1.2m and a height-diameter ratio of 6.7, had a flow guider 7 in it. The flow guider 7 had a diameter of 110mm and a height of 600mm. The flow guider separated the reactor into a central flow area (area A) and a circular flow area (area B) (as shown in Fig. 3). Six nozzles were uniformly distributed on the circular cross section of area A at the bottom of the reactor. This reactor can be used as an airlift enzyme reactor, to prepare biodiesel through transesterification reaction between animal and plant oils and methanol in the presence of lipase. This reaction system contained volatile liquid methanol, and the vacuum level was controlled by gas pump, using volatilized methanol gas as the impetus for the circulation of liquids inside the reactor. This loop reactor can achieve the same mixing effects as using external gases as the circulation impetus.

Compared to common loop reactors with external gases injected, the airlift loop reactor with no external gases effectively reduces the consumption of external gases, and therefore has significant economic implications and good prospects for industrial applications.

## Claims

1. A process for the transesterification reaction between animal and plant oils and methanol in the presence of lipase, using an airlift loop reactor without the need for external gases, wherein the airlift loop reactor comprises a main reactor (1), a gas circulation line (2), a gas pump (3), a jacket (4), a gas inlet (5), a gas outlet (6), a flow guider (7), a gas flowmeter (8), a feed inlet (9), a discharge opening (10),
wherein the process comprises the steps of:
connecting the gas outlet (6) on top of the airlift loop reactor to the gas inlet (5) at the bottom of the reactor, and
installing the gas pump (3) on the gas circulation line (2), hence directly using internal gases as circulation impetus, so that gases are directed by the gas circulation line (2) back to the bottom of the reactor after flowing out from the top of the reactor and then re-ejected into the reactor by the gas pump (3) and used as impetus for liquid circulation again; and
wherein the internal gases include gases generated during the reaction, gases participated in the reaction and volatile components.

2. The process of claim 1, further comprising adjusting pressure through one-way valve (12) installed on a by-pass gas line (11) connected to the gas circulation line (2).

## Patentansprüche

1. Verfahren zur Umesterungsreaktion zwischen tierischen und pflanzlichen Ölen und Methanol in Gegenwart von Lipase, unter Verwendung eines Airlift-Schlaufenreaktors ohne die Notwendigkeit für externe Gase, wobei der Airlift-Schlaufenreaktor einen Hauptreaktor (1), eine Gaszirkulationsleitung (2), eine Gaspumpe (3), einen Mantel (4), einen Gaseinlass (5), einen Gasauslass (6), einen Strömungsführer (7), einen Gasdurchflusmesser (8), einen Zuführungseinlass (9), eine Auslassöffnung (10) umfasst,
wobei das Verfahren die Schritte umfasst:
Anschließen des Gasauslasses (6) auf der Oberseite des Airlift-Schlaufenreaktors an den Gaseinlass (5) auf der Unterseite des Reaktors, und
Installieren der Gaspumpe (3) an der Gaszirkulationsleitung (2), also direkt unter Verwendung von internen Gasen als Zirkulationsimpuls, so dass Gase nach dem Abfluss von der Oberseite des Reaktors durch die Gaszirkulationsleitung (2) zurück zum Boden des Reaktors geleitet werden und dann von der Gaspumpe (3) wieder in den Reaktor ausgestossen werden und
als Impuls für die Flüssigkeitszirkulation wieder verwendet; und
wobei die internen Gase Gase umfassen, die während der Reaktion erzeugt werden, Gase die an der Reaktion teilgenommen haben und volatile Komponenten.

2. Verfahren nach Anspruch 1, weiter umfassend die Einstellung des Drucks durch ein Einwegventil (12), das auf einer Bypass-Gasleitung (11) installiert ist, die mit der Gaszirkulationsleitung (2) verbunden ist.

## Revendications

1. Procédé pour la réaction de transestérification entre des huiles animales et végétales et du méthanol en présence de lipase, à l'aide d'un réacteur à boucle par circulation d'air sans recourir à des gaz externes, dans lequel le réacteur à boucle par circulation d'air comprend un réacteur principal (1), une conduite de circulation de gaz (2), une pompe à gaz (3), une chemise (4), un orifice d'entrée de gaz (5), un orifice de sortie de gaz (6), un dispositif de guidage d'écoulement (7), un débitmètre de gaz (8), un orifice d'entrée d'alimentation (9), une ouverture d'évacuation (10),
dans lequel le procédé comprend les étapes de :
raccordement de l'orifice de sortie de gaz (6) sur le dessus du réacteur à boucle par circulation d'air à l'orifice d'entrée de gaz (5) au fond du réacteur, et
installation de la pompe à gaz (3) sur la conduite de circulation de gaz (2), utilisant ainsi directement les gaz internes en tant qu'impulsion de circulation, de sorte que des gaz soient dirigés par la conduite de circulation de gaz (2) en retour vers le fond du réacteur après être sortis du dessus du réacteur puis ré-éjectés dans le réacteur par la pompe à gaz (3) et utilisés en tant qu'impulsion pour la circulation de liquide à nouveau ; et
dans lequel les gaz internes comportent des gaz générés pendant la réaction, des gaz ayant participés à la réaction et des composants volatils.

2. Procédé selon la revendication 1, comprenant en outre le réglage de pression par l'intermédiaire d'une soupape de non-retour (12) installée sur une conduite de gaz de dérivation (11) raccordée à la conduite de circulation de gaz (2).
